# EUROPEAN PATENT SPECIFICATION

(11) **EP 2 046 306 B1**
(45) Date of publication and mention of the grant of the patent: **18.04.2012**
(21) Application number: 07789340.2
(22) Date of filing: 20.07.2007
(51) Int. Cl.: A61K 9/50, A61K 9/28

(54) **COATING COMPOSITION COMPRISING STARCH**
STÄRKE ENTHALTENDE BESCHICHTUNGSZUSAMMENSETZUNG
COMPOSITION DE REVÊTEMENT CONTENANT DE L'AMIDON

(30) Priority: 27.07.2006 GB 0614933; 01.09.2006 GB 0617215
(43) Date of publication of application: 15.04.2009
(73) Proprietor: University of Sunderland, Sunderland Durham SR1 3SD (GB)
(72) Inventor: PODCZECK, Gisela Fridrun, Houghton-le-Spring DH4 5FB (GB); FREIRE, Ana Cristina Teixeira de Almeida Lopes, 3150-152 Condeixa-a-nova (PT)
(74) Representative: Harrison Goddard Foote
(86) International application number: PCT/GB2007/050428
(87) International publication number: WO 2008/012573

(56) References cited:
- WO-A-99/52512
- WO-A-2007/092717
- US-B1- 6 703 044
- US-B1- 6 951 657
- MILOJEVIC S. ET AL.: 'Amylose as a coating for drug delivery to the colon: Preparation and in vitro evaluation using 5-aminosalicylic acid pellets' JOURNAL OF CONTROLLED RELEASE vol. 38, 1996, pages 75 - 84, XP004037421
- ROWE R.C. ET AL: 'Handbook of Pharmaceutical Excipients, 6th ed.', 2006 article 'Polymethacrylates', pages 525 - 535
- AUTON M.E.: 'Pharmaceutics: The Science of Dosage Form Design, 2nd ed.', 2002 article CHAPTER 28: COATING OF TABLETS AND MICROPARTICULATES, pages 441 - 448
- VOIGT R.; BORNSCHEIN M.: 'Lehrbuch der pharmazeutischen Technologie, 6. Auflage', 1987 pages 290 - 291
- FREIRE CRISTINA ET AL: "Assessment of the in-vivo drug release from pellets film-coated with a dispersion of high amylose starch and ethylcellulose for potential colon delivery", JOURNAL OF PHARMACY AND PHARMACOLOGY, vol. 62, no. 1, January 2010 (2010-01), pages 55-61, ISSN: 0022-3573

## Description

### Field of the Invention

This invention relates to a coating composition and its use, e.g in the preparation of formulations for colonic drug delivery.

### Background to the Invention

Colonic diseases and conditions, for example inflammatory bowel disease, colorectal cancer, constipation and infections, are poorly managed by both orally administered drugs, which are mainly absorbed in the stomach and small intestine, and rectally administered drugs, which rarely succeed in the reaching the ascending colon. Colonic drug delivery has been used to overcome these limitations. Any system that specifically targets drugs into the colon has obvious advantages of reducing the oral dose required and cutting down important side effects of drugs such as corticosteroids.

Oral administration of vaccines, proteins and therapeutic peptides into the systemic circulation is also limited by significant degradation in the acidic environment of the stomach and enzymatic degradation in the small intestine. In contrast, the colon has lower enzymatic activity and near neutral pH and so has been used as a portal for the entry of these substances into the systemic circulation.

Targeting active compounds into the colon can be achieved using time-dependent systems, pH-dependent systems or systems based on the enzymatic activity of the colon. The latter seem to provide high specificity at the site of drug release since they rely on the specific breakdown of biodegradable polymers by colonic microflora. These biodegradable polymers, mainly polysaccharides, can be part of the coating or simply a matrix forming material. The coating systems conveniently allow a higher drug content.

Coating compositions based on glassy and crystalline forms of amylose are known. The glassy form of amylose is believed to resist degradation by the pancreatic α-amylases but still undergo fermentation by colonic enzymes. The compositions generally comprise an aqueous dispersion of amylose. Since amylose is swollen by aqueous media, the compositions also comprise a substance which controls swelling of the amylose. Often, this substance is a water-insoluble polymer such as ethyl cellulose.

Milojevic et al (J. Control. Release, 38, 75-84, 1995) describe a complex procedure for the extraction of amylose from smooth seeded pea starch which ultimately yields an amylose-butan-1-ol complex. An aqueous dispersion of this amylose complex is then mixed with an aqueous ethyl cellulose dispersion at high temperatures (generally 70 °C) in different ratios w/w. These temperatures are necessary in order to melt the complex and obtain an amylose solution. Formation of a glassy or crystalline amylose film depends on the rate of drying during the film coating process.

US 6534549 describes an amylose coating composition formed by mixing an aqueous dispersion of an amylose-butan-1-ol complex with a solution of ethyl cellulose dissolved in a water-miscible organic solvent, such as ethyl lactate. In these dispersions, the ratios of amylose to ethyl cellulose are in the range of 1:2 and 3:2 and the solvent system contains at least 50% w/w organic solvent. Generally, the temperatures used in this process do not need to be higher than 60 °C and can often be ambient temperature.

US 2005/0220861 describes the use of a composition comprising glassy amylose, ethyl cellulose and dibutyl sebacate to coat pellets containing prednisolone sodium metasulphobenzoate. The composition is obtained by heating an aqueous or aqueous/alcohol solution of glassy amylose mixed with an aqueous ethyl cellulose dispersion plasticized with dibutyl sebacate, to obtain amylose in amorphous form.

Although amylose has been used in various coating compositions, it must be used in a glassy or amorphous form, which requires lengthy and complex processing. There remains a need for coating compositions which provide specific release in the colon and which can be simply and readily prepared.

### Summary of the Invention

The present invention is based at least in part on a discovery that coating compositions comprising starch are particularly suited for use in the colonic delivery of therapeutic and diagnostic agents. Starch is specifically broken down by colonic microflora, making it ideal for the delivery of drugs into the colon. Also, unlike amylose, starch does not require processing to make it glassy or amorphous. In order to avoid release of the active agent before reaching the colon, a composition of the invention further comprises an agent that aids formation of a film and controls swelling of the starch.

Accordingly, in a first aspect of the invention there is provided a coating composition comprising an aqueous medium and, dispersed therein, starch which comprises at least 40% amylose and a film-forming agent which is a substantially water-insoluble polymer which controls swelling of the starch by the aqueous medium.

In a second aspect, the invention provides a formulation which is coated with a composition of any preceding claim.

A further aspect is a method of treating or diagnosing a disease or condition of the colon, which comprises administering a formulation of the invention.

The invention also provides a process for producing a composition of the invention, which comprises admixing an aqueous medium, starch and a film-forming agent which controls swelling of the starch by the aqueous medium.

The invention also provides a method of treatment of local diseases associated with the colon comprising administering a formulation of the invention. By way of example, local diseases include Crohn's disease, ulcerative colitis, colorectal cancer and amebiasis.

A coating composition of the invention provides specificity in the site of drug release. A formulation of the invention may be used with a wide variety of active ingredients, either for the local treatment of colonic diseases and conditions or the delivery of proteins and peptides and vaccines intended to be absorbed by the colonic mucosa. Thus, the invention may find utility in the therapy of inflammatory bowel disease, colorectal cancer, constipation and infection. Coating thicknesses may be varied to target, for example, drug release or absorption in the terminal small intestine or the proximal, transversal or terminal colon. A formulation of the invention may allow controlled release of one or more active ingredients.

### Brief Description of the Drawings

Fig. 1 shows the drug release from uncoated pellets and pellets coated with Hylon^{®} VII (total weight gain of 8, 16 and 24%) plasticized with 10 % w/w of dibutyl sebacate at pH 1.2.
Fig. 2 shows the drug release from pellets coated with a composition comprising Hylon^{®} VII (total weight gain of 8, 16 and 24%) and 10 % w/w of dibutyl sebacate at pH 7.2.
Fig. 3 shows the drug release from the pellets coated with a composition comprising Hylon^{®} VII-Surelease^{®} in a ratio of 1:4 and 1:5 with different coating weight gains at pH 1.2.
Fig. 4 shows the drug release from the pellets coated with a composition comprising Hylon^{®} VII and Surelease^{®} in a ratio of 1:4 and 1:5 with different coating weight gains at pH 7.2.
Fig. 5 shows the drug release from pellets coated with a composition comprising Hylon^{®} VII/Hylon^{®} V and Surelease^{®} in a ratio of 1:5 (total weight gain of 24 %) at pH 1.2 and pH 7.2.
Fig. 6 shows the drug release from pellets coated with Surelease^{®} (total weight gain of 25 %) at pH 1.2 and pH 7.2.
Fig. 7 shows the drug release from pellets coated with Hylon^{®} VII and Surelease^{®} in a ratio of 1:2 and 1:5 (total weight gain of 30% and 24 %, respectively) in simulated gastric juice.
Fig. 8 shows the drug release from pellets coated with Hylon^{®} V and Surelease^{®} in a ratio of 1:2 and 1:5 (total weight gain of 30% and 24 %, respectively) in simulated gastric juice.
Fig. 9 shows the drug release from pellets coated with Hylon^{®} VII and Surelease^{®} in a ratio of 1:2 and 1:5 (total weight gain of 30% and 24 %, respectively) in simulated intestinal juice.
Fig. 10 shows the drug release from pellets coated with Hylon^{®} V and Surelease^{®} in a ratio of 1:2 and 1:5 (total weight gain of 30% and 24 %, respectively) in simulated intestinal juice.
Fig. 11 shows the drug release from pellets coated with Hylon^{®} VII/Hylon^{®} V and Surelease^{®} in a ratio of 1:2 and 1:5 (total weight gain of 30% and 24 %, respectively), for 2 hours in simulated gastric juice (SGJ), followed by 4 hours in simulated intestinal fluid (SIF) and 18 hours in simulated colonic fluid (SCF).
Fig. 12 shows the drug release from pellets coated with Hylon^{®} VII or Hylon^{®} V and Surelease^{®} in a ratio of 1:2 and 1:5 (total weight gain of 30% and 24 %, respectively), for 4 hours in simulated intestinal fluid (SIF) followed by 20 hours in simulated colonic fluid (SCF).

### Description of Various Embodiments

A coating composition of the invention comprises an aqueous medium and, dispersed therein, starch which comprises at least 40% amylose and a film-forming agent which is a substantially water-insoluble polymer which controls swelling of the starch by the aqueous medium.

The aqueous medium may be any suitable medium known in the art, and may optionally comprise a non-aqueous (e.g. an organic) medium. In a particular embodiment, the medium is water or a water/alcohol (e.g. water/ethanol) mixture.

Dispersed in the aqueous medium is a starch which comprises at least 40% amylose. The term "starch" as used herein includes reference to a carbohydrate comprising amylose and amylopectin. A wide range of starches with different amylose:amylopectin ratios can be used. The starch may comprise at least 50 % amylose, more particularly at least 60 % amylose and especially at least 70 % amylose. Examples of suitable starches include Hylon V^{®} (a corn starch containing about 70 % amylose) and Hylon VII^{®} (a corn starch containing about 50 % amylose), both of which are available from National Starch and Chemical Company.

Starch generally undergoes a significant degree of swelling in the presence of aqueous media. If the starch is too swollen, the active ingredient may be released before the formulation reaches the colon. A coating composition of the invention therefore also comprises a film-forming agent which is a substantially water-insoluble polymer which controls swelling of the starch. This agent is typically one that inhibits swelling of the starch, but there may be circumstances where the use of an agent which promotes swelling of the starch is desirable. Of particular mention are polymers which are substantially water-insoluble, a particular example being ethyl cellulose and low permeability polymethacrylates (e.g. Eudragit^{®} RS or similar). The agent, e.g. ethyl cellulose, may be used in the form of a dispersion, especially an aqueous dispersion.

The composition may further comprise a plasticizer in order to facilitate formation of the coating, control porosity and improve the mechanical properties of the coating. Many different plasticizers are known, examples including di-and tricarboxy acid esters such as triethyl citrate, glycerol triacetate, acetyl tributyl citrate, tributyl citrate, triacetin and dibutyl sebacate. Of particular mention is dibutyl sebacate.

The coating composition can be prepared simply by admixture of the aqueous medium, starch and the film-forming agent.

By way of illustration, a coating composition of the invention may be prepared as follows. Starch is first dispersed in water or another aqueous medium, to form a starch dispersion. This step may be carried out at a temperature ranging from about room temperature to about 100 °C, e.g. about 75 to about 85 °C, particularly about 80 °C. The resulting dispersion is allowed to cool where necessary (e.g. to at least from about 60 to about 70 °C). A film-forming agent is then added to the starch dispersion. This step may be achieved by adding the film-forming agent in the form of a dispersion, e.g. an aqueous dispersion. Of mention is a dispersion of ethyl cellulose in water or a water/ethanol mixture. In one embodiment, an aqueous dispersion comprising the film-forming agent and a plasticizer is added to the starch dispersion. In this regard, an aqueous dispersion comprising ethyl cellulose and dibutyl sebacate may be used. Of mention are aqueous dispersions containing ethyl cellulose, dibutyl sebacate and one or more stabilizers (e.g. Surelease^{®} dispersions from Colorcon).

The coating composition may then be applied to a pharmaceutical or other (e.g. diagnostic) formulation. Suitable coating processes, for example, fluidized bed and coating pan processes, are well known in the art. A particular advantage of the invention is that the coating does often not need to be cured after application.

The thickness of the coating also influences the rate at which an active material is released from a dosage form. Depending upon the solubility of the active material in the dosage form, release is generally slower when thicker coatings are employed. Thicker coatings are also required when highly soluble active materials are employed in order to prepare a dosage form in which the release of active material in the colon can be adequately controlled.

A suitable thickness can be arrived at by routine experimentation but, by way of guidance, it may be stated that a thickness in the range of from about 10 to about 60 µm is often preferred, especially in the range of about 20 to about 50 µm. In many cases, the coating should be at least 30 µm thick. Of particular mention are coating thicknesses of from about 30 to about 60 µm, e.g. from about 40 to about 50 µm. The thickness of the coating can be optimised to ensure specific release of the active ingredient at the target site. Varying the coating thickness may also allow targeting of active compounds of differing solubilities into the colon.

Coat thickness can also be defined as the total weight gain, i.e. the percentage of weight increase of the formulation upon coating. This is normally in the range of 10 to 40%. In particular, the coating may be applied to a total weight gain of from about 15 to about 40 %, more preferably from about 20 to about 35 %.

The dissolution profile of the active ingredient can also be controlled by varying the proportion of the components present in the coating composition. In this way, drug release can be minimised in the upper gastrointestinal tract and maximised in the colon. In many cases, it will be desirable for the composition to include relatively minor and major proportions of the starch and the film-forming agent. Thus, for example, the ratio of starch to film-forming agent may range from about 1:1 to about 1:6. Particular ratios range from about 1:2 to about 1:5, e.g. from about 1:4 to about 1:5.

The strongly acidic gastric fluid may interact with the coating, making it difficult for the starch to be digested. In this case, it may be desirable to apply a further, enteric coating, which inhibits dissolution of the formulation in the stomach. Any suitable enteric coating known in the art may be used. Of mention are enteric coatings that dissolve at pH 5.5 or more. Of particular mention are enteric coatings comprising an aqueous dispersion of methacrylic acid-ethyl acrylate copolymers (e.g. Kollicoat MAE 30 DP).

A formulation of the invention is typically in a solid form suitable for oral administration. Suitable forms include pellet, tablet, capsule, powder, granule and extrudate forms. Pellets may be substantially spheroidal, typically having a diameter of from about 0.1 mm to about 5.0 mm, e.g. from about 0.5 mm to about 5.0 mm, in particular from about 0.5 mm to about 2.5 mm, more particularly from about 0.8 to about 1.5 mm.

The formulation may comprise one or more active ingredients. The term "active ingredient" as used herein includes reference to foodstuffs, pharmaceuticals and other therapeutic agents, diagnostic agents, and electrically conducting components, without limitation. Of particular mention are compounds or compositions useful in human or veterinary medicine in therapy or diagnosis. Therapeutic agents include pharmaceuticals, vaccines, proteins and therapeutic peptides. These agents may be absorbable from the colon or useful in the local treatment of colonic diseases. Of mention are aminosalicylates (e.g. balsalazide, mesalazine, olsalazine and sulfasalazine) and corticosteroids (e.g. hydrocortisone, budesonide and prednisolone). Alternatively or additionally, the formulation may comprise one or more diagnostic agents. Diagnostic agents include, for example, those suitable for use in X-ray and NMR imaging techniques. An alternative diagnostic area lies in the delivery of potentially allergenic foodstuff components to the colon for the diagnosis of allergies. The or each active agent is typically present in a core coated with a composition of the invention.

A formulation of the invention may comprise an active ingredient coated with starch and a swelling control agent and adapted for swelling of the starch in the upper tract to be resisted or controlled in a manner functional to allow release of useful amounts of the active ingredient in the colon. The formulation typically has a coating which impedes contacting the starch with fluid in the upper (pre-colonic) GI tract. In one embodiment, suitably, the formulation contains further diluents, excipients and/or carriers.

The invention may have particular application in the manufacture of medicaments which comprise for example peptides, proteins and nucleic acid molecules e.g. oligonucleotides e.g. vaccines. Oral delivery of proteins, peptides and nucleic acid molecules e.g. oligonucleotides represents a challenge due to the biological barriers that restrict protein and peptide absorption from the gastrointestinal (GI) tract, which include pH variability, enzymatic degradation, and membrane efflux. Thus, the present invention provides compositions which can be targeted to the colon, which is less proteolytically active than other parts of the GI tract and so may be used in particular for peptide, protein or nucleic acid molecule based drugs. Thus, in one embodiment of the present invention, the composition comprises an active ingredient selected from a peptide, a protein, a nucleic acid molecule e.g. oligonucleotide or combination thereof.

A particular active ingredient is 5-aminosalicylic acid (5-ASA), a drug which is used orally in the treatment of colonic disorders. When free 5-ASA is administered orally, little of the drug reaches the colon as the stomach and small intestine inactive and/or absorb the drug. The present invention provides a composition comprising 5-ASA which can be administered orally with delayed release of a substantial amount of the active ingredient in the colon. The 5-ASA is preferably provided in the form of spherules, suitably spheronized in admixture with microcrystalline cellulose, and a minor proportion of an inorganic binder such as bentonite may be added, but is not essential.

Actual dosage levels of the or each active ingredient in the formulation may be varied so as to obtain an amount effective to achieve the desired therapeutic response for a particular patient, compositions, and mode of administration. The selected dosage level will depend upon the activity of the particular compound, the route of administration, the severity of the condition being treated and the condition and prior medical history of the patient being treated. However, it is within the skill of the art to start doses of the compound at levels lower than required for to achieve the desired therapeutic effect and to gradually increase the dosage until the desired effect is achieved.

An exemplary dosage level of active ingredient is from about 0.01 to about 500 mg per kg patient body weight per day which can be administered in single or multiple doses. In particular, the dosage level may be from about 0.1 to about 250 mg/kg per day, e.g. about 0.5 to about 100 mg/kg per day. A suitable dosage level may be about 0.01 to 250 mg/kg per day, about 0.05 to 100 mg/kg per day, or about 0.1 to 50 mg/kg per day. Within this range the dosage may be 0.05 to 0.5, 0.5 to 5 or 5 to 50 mg/kg per day. For oral administration, the formulations may contain 1.0 to 1000 milligrams of the active ingredient, particularly 1.0, 5.0, 10.0, 15.0, 20.0, 25.0, 50.0, 75.0, 100.0, 150.0, 200.0, 250.0, 300.0, 400.0, 500.0, 600.0, 750.0, 800.0, 900.0 and 1000.0 milligrams of the active ingredient for the symptomatic adjustment of the dosage to the patient to be treated. The formulation may be administered on a regime of 1 to 4 times per day, preferably once or twice per day. The dosage regime may be adjusted to provide the optimal therapeutic response.

It will be appreciated that the or each active ingredient may be mixed with other carrier materials suitable to its particular use. Thus, the formulation, especially the core thereof, may further comprise one or more of: a) fillers or extenders such as starches, lactose, sucrose, glucose, mannitol and silicic acid; b) binders such as carboxymethylcellulose, alginates, gelatin, polyvinyl pyrrolidone, sucrose and acacia; c) humectants such as glycerol; d) disintegrating agents including inorganic compound such as iron oxides, barium sulphate and calcium carbonate; e) solution retarding agents such as waxes; f) absorption accelerators such as quaternary ammonium compounds; g) wetting agents such as cetyl alcohol and glycerol monostearate; h) absorbents such as kaolin and bentonite clay and i) lubricants such as talc, calcium stearate, magnesium stearate, solid polyethylene glycols, sodium lauryl sulfate and mixtures thereof. The formulation may further comprise a buffering agent.

The invention further includes use of a starch in the manufacture of a medicament or a diagnostic formulation for release of the active ingredient(s) in the colon.

In one embodiment, the medicament comprises a peptide, a protein or a nucleic acid molecule e.g. an oligonucleotide or a combination thereof. Preferably, the medicament comprises an active ingredient which is ordinarily broken down in the upper GI tract, prior to reaching the colon.

In one embodiment, the medicament is for treating a disorder which is a local disease of the colon. By way of example, the local disease may be selected from Crohn's diseases, ulcerative colitis, colorectal cancer and amebiasis.

It will be appreciated that the subject matter of the invention includes, but is not limited to, pharmaceutical and diagnostic formulations having a coating comprising starch as described above, in which the starch is not glassy or amorphous but is partially or substantially crystalline, e.g. partially crystalline

Also included is a method for protecting a pharmaceutical or diagnostic formulation against enzymes of the upper GI tract while allowing attack by colonic enzymes, comprising applying to the formulation a coating material comprising a starch.

The following Examples illustrate the invention.

### Example 1: Preparation of coated pellets

### Preparation of drug-loaded pellets

Pellets containing 50 % of the model drug 5-aminosalicylic acid (5-ASA) and microcrystalline cellulose (Avicel^{®} PH 101) were produced by an extrusion-spheronisation process. 5-ASA, microcrystalline cellulose (50:50 w/w) and distilled water as needed were mixed to form a wet mass, which was then extruded and spheronized to form pellets. The pellets were then dried on a porous tray at room temperature for a period of 48 hours and sieved to obtain pellets with a size range of 1.00 to 1.40 mm.

### Preparation of starch coatings

Hylon^{®} VII was dispersed in water and heated at 80 ± 5 °C. The attained dispersion was plasticized with 10 % w/w dibutyl sebacate corresponding to the dry weight of the starch. Various coating thicknesses were tried by varying the amount of starch in the coating dispersion.

### Preparation of starch-ethyl cellulose coatings

Hylon^{®} VII or Hylon^{®} V starch was dispersed in water under constant stirring and heated at a temperature of 80± 5 °C. The resulting dispersion was allowed to cool and an ethyl cellulose (Surelease^{®}) dispersion added under constant stirring until the dispersions were completely mixed. Coatings with varying thicknesses were prepared by altering the amount of Hylon^{®} VII in the coating dispersion. Dispersions comprising 1 part of Hylon^{®} VII/Hylon^{®} V and 4 or 5 parts of ethyl cellulose dispersion (Surelease^{®}) plasticized with 10 % dibutyl sebacate (corresponding to the dry weight of the polymers) were prepared.

### Preparation of ethyl cellulose coatings

Ethyl cellulose dispersions were prepared from Surelease^{®} under the same conditions as the starch-ethyl cellulose dispersions, by admixing the same amount of distilled water at 70°C.

### Coating of pellets

A Wurster fluid bed coating procedure was used to coat the 5-ASA pellets with the dispersions described above. The dispersions were maintained at 70 °C throughout the coating process. The coating parameters were: batch size 30 g, inlet temperature of 60 °C, product temperature of 45-50 °C, outlet temperature of 40-45 °C, flow rate of 0.7-0.8 ml/min., nozzle diameter 1.00 diameter and atomization air pressure of 2.2 to 2.4 bar. The coated pellets were dried in a fluid bed coated for 10 minutes at 60 °C. No curing of the film coatings was necessary.

### Example 2: Drug release study

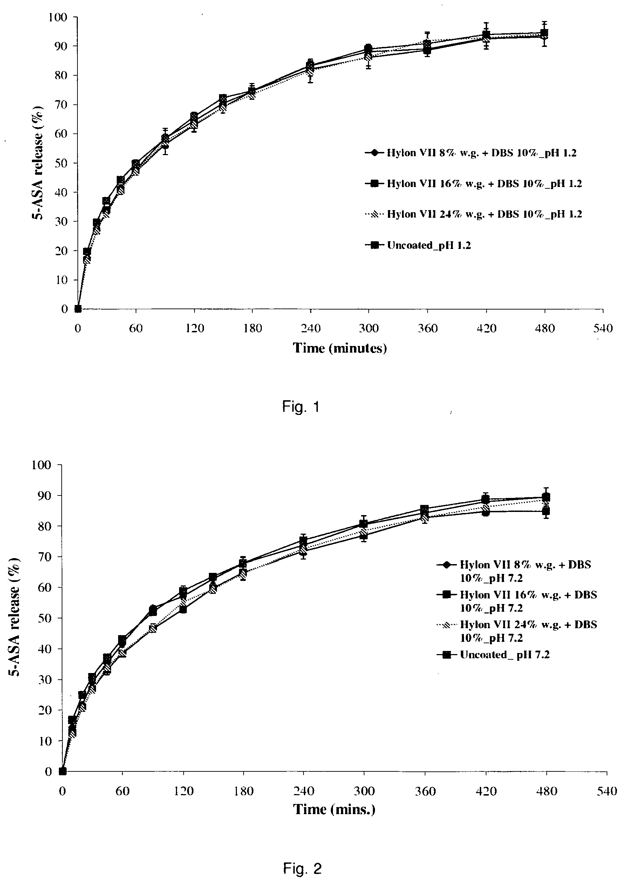

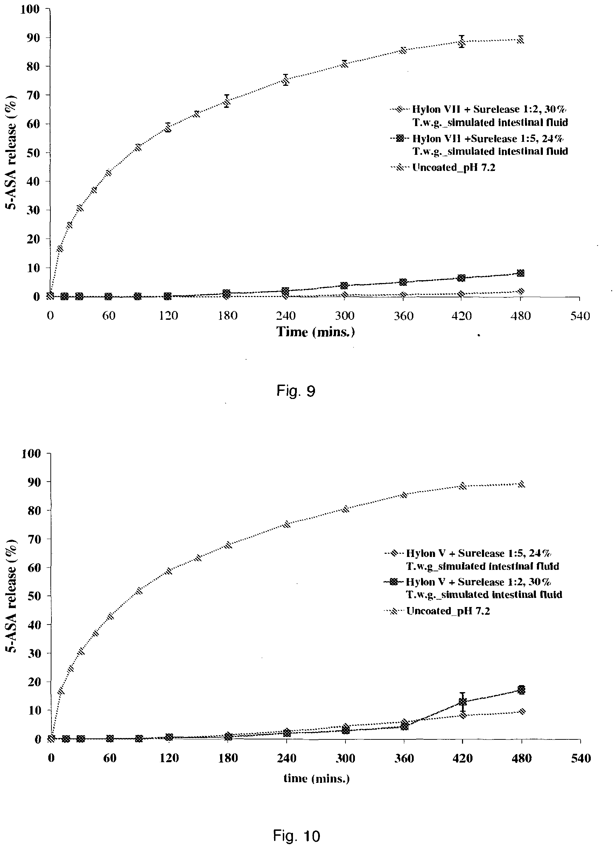

Release of 5-ASA from the coated pellets of Example 1 was measured using a dissolution tester by a USP paddle method. All tests were conducted in triplicate, in 900 ml of dissolution media maintained at 37± 1 °C with a paddle rotation speed of 100 rpm. The dissolution media used were: (a) 0.1 N HCl (pH 1.2), (b) phosphate buffer (pH 7.2), (c) simulated gastric fluid, (d) simulated intestinal fluid and (e) simulated colonic fluid. The dissolution profiles are shown in Figs. 1 to 12.

### Pellets coated with starch

Figs. 1 and 2 show the dissolution profiles of the pellets coated with starch and a plasticizer at pH 1.2 and pH 7.2 respectively. These pellet formulations released a significant amount of drug under these conditions, due to the high degree of swelling of the starch by the aqueous solvent.

### Pellets coated with ethyl cellulose

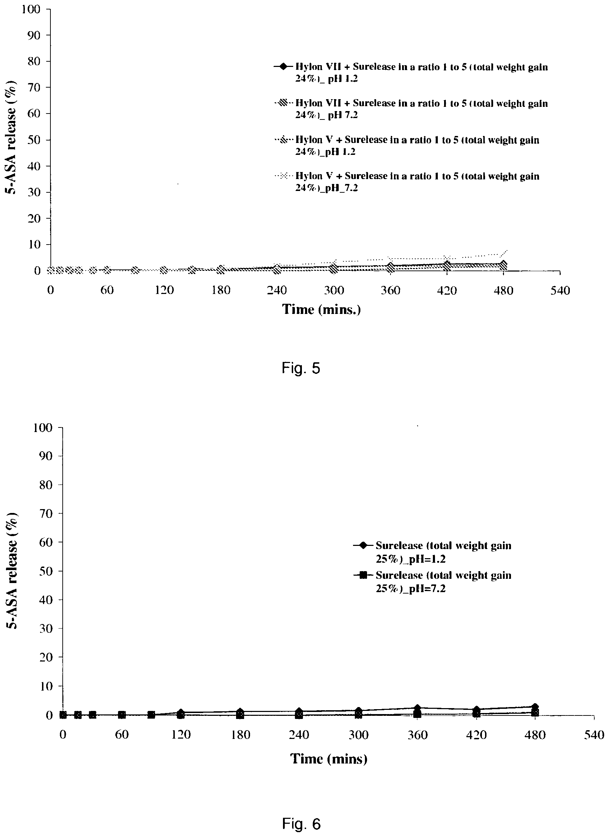

Fig. 6 shows the dissolution profile of pellets coated with ethyl cellulose. This figure illustrates that ethyl cellulose films without starch do not release any drug.

### Pellets coated with starch and ethyl cellulose

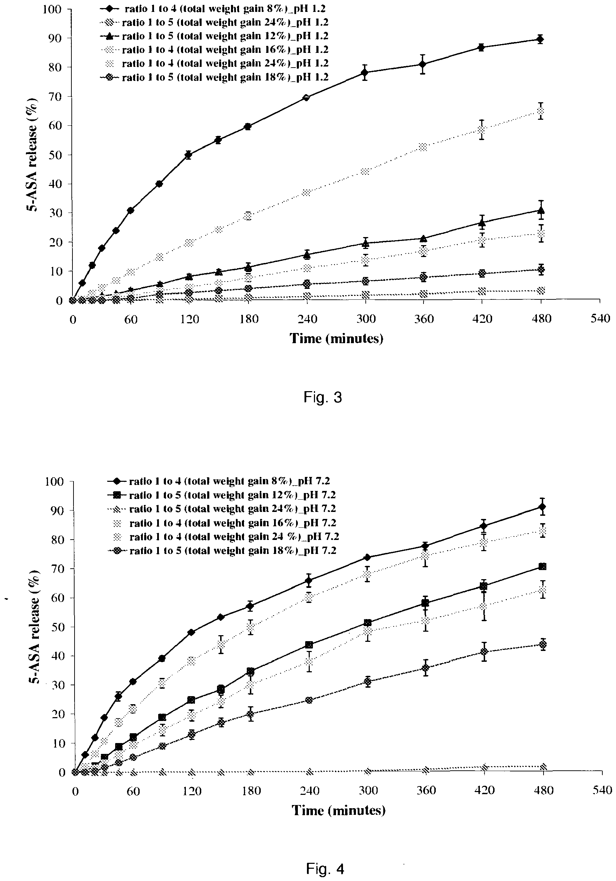

Figs. 3 and 4 show the dissolution profile of pellets coated with starch, ethyl cellulose and plasticizer at pH 1.2 and pH 7.2 respectively. The effect of the ratio of starch: ethyl cellulose and the total weight gain were assessed. Increasing the amount of ethyl cellulose relative to the amount of starch was found to significantly reduce the dissolution rate. Increasing the total weight gain had a similar effect. The coating having a starch:ethyl cellulose ratio of 1:5 and a total weight gain of 24 % resulted in the lowest rate of drug dissolution at each pH tested.

Fig. 5 shows the effect of using starch having varying amounts of amylose.

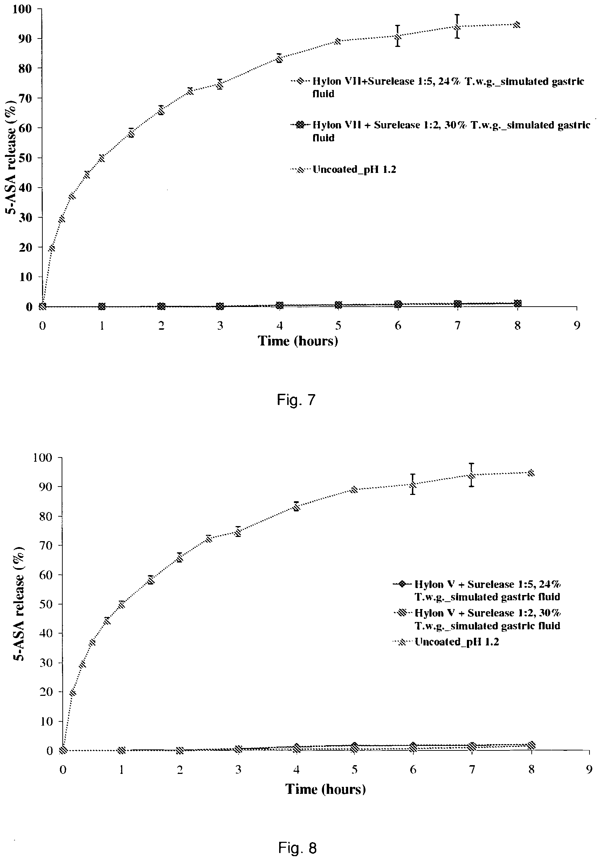

Selected formulations were further assessed in simulated gastric juice (SGJ; B.P. 2005, appendix XII-B-A187) and simulated intestinal fluid (SIF), which contained pepsin and pancreatin respectively. As Figs. 7 to 10 show, only negligible drug release was observed in these fluids over the 8-hour period tested.

The colonic environment was then simulated using *Bacillus Licheniformis* α-amylase (the concentration of α-amylase was varied from 50 U/ml to 500 U/ml) in phosphate buffer pH 7.2. In this test, pellets were maintained in simulated gastric juice (SGJ) for 2 hours, followed by 4 hours in simulated intestinal juice (SIJ) and 18 hours in colonic simulated fluid (SCF) or 4 hours in SIJ followed by 20 hours in SCF in order to assess the influence of the acid on the coating formulation.

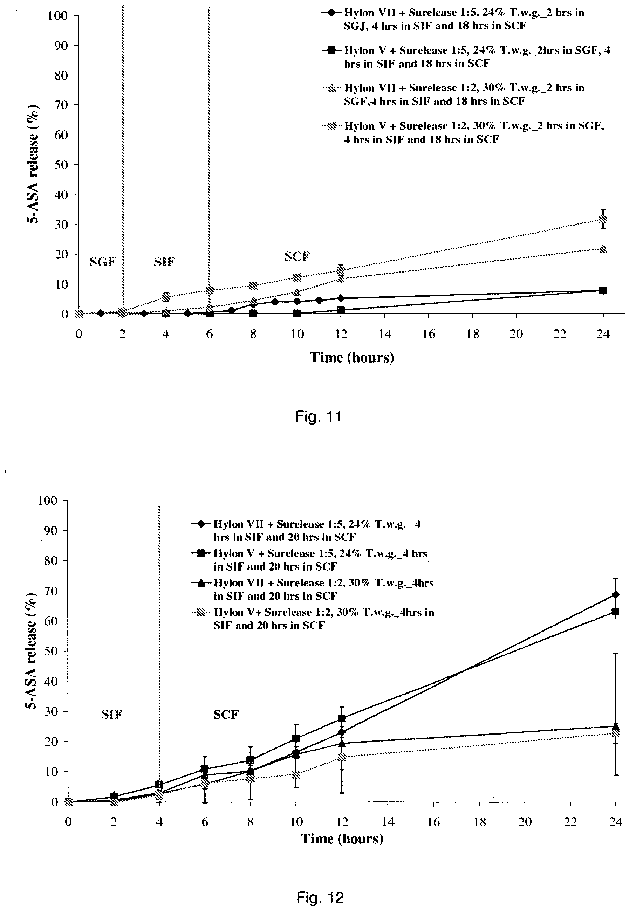

Fig. 11 shows the effect of transferring a particular pellet formulation from SGF, to SIF and then to simulated colonic fluid (SCF). When pellets coated with Hylon^{®} VII/Hylon^{®} V and Surelease^{®} in a ratio of 1 to 5 (total weight gain of 24 %) were placed in SGF and then transferred to SIF, drug release was suppressed.

As Fig. 12 shows, a different profile was observed when the mentioned formulations were placed in SIF followed by the 20 hours in SCF. In this case, drug release in SCF was high compared with that of Fig. 11, suggesting that some kind of interaction between the components of the coating and the lower pH of the SGF might occur.

In summary, the coating dispersion comprising 1 part of Hylon VII/Hylon V and 5 parts of Surelease^{®} in a total weight gain of 24 % suppressed the drug release in the simulated conditions of the stomach and small intestine. In the presence of colonic enzymes, the release of 5-ASA was high, making this system feasible for the delivery of drugs into the colon.

## Claims

1. A coating composition comprising an aqueous medium and, dispersed therein, starch which comprises at least 40% amylose and a film-forming agent which is a substantially water-insoluble polymer which controls swelling of the starch by the aqueous medium.

2. A composition according to claim 1, wherein the aqueous medium is water, or comprises water and an alcohol (e.g. ethanol).

3. A composition according to any preceding claim, wherein the starch comprises from 40 to 80 % amylose.

4. A composition according to any preceding claim, wherein the starch comprises from 50 to 70 % amylose.

5. A composition according to any preceding claim, wherein the film-forming agent inhibits swelling of the starch by the aqueous medium.

6. A composition according to any preceding claim, wherein the film-forming agent comprises ethylcellulose.

7. A composition according to any preceding claim, wherein the ratio of starch to film-forming agent is from 1:1 to 1:6.

8. A composition according to any preceding claim, which further comprises a plasticizer, wherein optionally the plasticizer is dibutyl sebacate.

9. A formulation for colonic drug delivery comprising a core comprising an active ingredient and a coating which comprises starch which comprises at least 40% amylose and a film-forming agent which is a substantially water insoluble polymer which controls swelling of the starch by an aqueous medium.

10. A formulation according to claim 9 which is obtainable by coating a core comprising an active ingredient with a composition of any of claims 1 to 8.

11. A formulation according to claim 9 or claim 10, wherein the active ingredient is a pharmaceutical, vaccine, protein or a peptide, wherein optionally said active ingredient is a diagnostic agent.

12. A formulation according to any of claims 9 to 11, which further comprises an outer, enteric coating, wherein optionally the enteric coating comprises a copolymer obtainable by polymerisation of monomers including methacrylic acid and ethyl acrylate.

13. A formulation according to any of claims 9 to 12, for use in therapy or diagnosis, and is optionally for use in the therapy or diagnosis of a colonic disease or condition.

14. A formulation according to any of claims 9 to 13, which is a controlled release formulation.

15. A formulation according to any of claims 9 to 14, wherein the active agent is an aminosalicylate or a corticosteroid.

16. A process for producing a composition according to any of claims 1 to 8, which comprises admixing an aqueous medium, starch which comprises at least 40% amylose and a film-forming agent which is a substantially water-insoluble polymer which controls swelling of the starch by the aqueous medium, wherein optionally the method comprises mixing a dispersion comprising starch with a dispersion containing said film-forming agent.

17. A process according to claim 16 wherein the method further comprises coating a formulation with said composition.

18. A formulation of claim 15 for use in the therapy or diagnosis of a disease or condition of the colon.

## Patentansprüche

1. Überzugszusammensetzung, die ein wässriges Medium umfasst sowie darin dispergierte Stärke, die mindestens 40 % Amylose und einen Filmbildner, bei dem es sich um ein im Wesentlichen wasserunlösliches Polymer handelt, welches das Quellen der Stärke durch das wässrige Medium regelt.

2. Zusammensetzung nach Anspruch 1, wobei es sich bei dem wässrigen Medium um Wasser handelt, oder wobei es Wasser und einen Alkohol (z.B. Ethanol) umfasst.

3. Zusammensetzung nach einem der vorstehenden Ansprüche, wobei die Stärke zwischen 40 und 80 % Amylose umfasst.

4. Zusammensetzung nach einem der vorstehenden Ansprüche, wobei die Stärke zwischen 50 und 70 % Amylose umfasst.

5. Zusammensetzung nach einem der vorstehenden Ansprüche, wobei der Filmbildner das Quellen der Stärke durch das wässrige Medium unterdrückt.

6. Zusammensetzung nach einem der vorstehenden Ansprüche, wobei der Filmbildner Ethylcellulose umfasst.

7. Zusammensetzung nach einem der vorstehenden Ansprüche, wobei das Verhältnis von Stärke zu dem Filmbildner zwischen 1:1 und 1:6 liegt.

8. Zusammensetzung nach einem der vorstehenden Ansprüche, wobei die Zusammensetzung ferner einen Plastifikator umfasst, wobei es sich bei dem Plastifikator optional um Dibutylsebacat handelt.

9. Rezeptur für die Darmarzneimittelverabreichung, welche einen Kern umfasst, der einen aktiven Bestandteil und einen Überzug umfasst, der Stärke umfasst, die mindestens 40 % Amylose umfasst und einen Filmbildner, bei dem es sich um ein im Wesentlichen wasserunlösliches Polymer handelt, welches das Quellen der Stärke durch ein wässriges Medium regelt.

10. Rezeptur nach Anspruch 9, die durch Überziehen eines Kerns, der einen aktiven Bestandteil umfasst, mit einer Zusammensetzung nach einem der Ansprüche 1 bis 8 erhalten werden kann.

11. Rezeptur nach Anspruch 9 oder Anspruch 10, wobei es sich bei dem aktiven Bestandteil um einen pharmazeutischen Stoff, einen Impfstoff, ein Protein oder ein Peptid handelt, wobei es sich bei dem aktiven Bestandteil optional um ein Diagnosemittel handelt.

12. Rezeptur nach einem der Ansprüche 9 bis 11, wobei diese ferner einen äußeren, magensaftresistenten Überzug handelt, wobei der magensaftresistente Überzug optional ein Copolymer umfasst, das durch Polymerisation von Monomeren erhalten werden kann, die Methacrylsäure und Ethylacrylat aufweisen.

13. Rezeptur nach einem der Ansprüche 9 bis 12 zum Einsatz in der Therapie oder Diagnose und optional zum Einsatz in der Therapie oder Diagnose einer Darmerkrankung oder eines Darmzustands.

14. Rezeptur nach einem der Ansprüche 9 bis 13, wobei es sich dabei um eine Rezeptur mit geregelter Abgabe handelt.

15. Rezeptur nach einem der Ansprüche 9 bis 14, wobei es sich bei dem aktiven Mittel um ein Aminosalicylat oder ein Corticosteroid handelt.

16. Verfahren zur Herstellung einer Zusammensetzung nach einem der Ansprüche 1 bis 8, wobei das Verfahren das Zusammenmischen eines wässrigen Mediums, Stärke, die mindestens 40 % Amylose und einen Filmbildner umfasst, bei dem es sich um ein im Wesentlichen wasserunlösliches Polymer handelt, welches das Quellen der Stärke durch das wässrige Medium regelt, wobei das Verfahren optional das Mischen einer Dispersion umfasst, die Stärke umfasst, mit einer Dispersion, die den genannten Filmbildner aufweist.

17. Verfahren nach Anspruch 16, wobei das Verfahren ferner das Überziehen einer Rezeptur mit der genannten Zusammensetzung umfasst.

18. Rezeptur nach Anspruch 15 zum Einsatz in der Therapie oder Diagnose einer Darmerkrankung oder eines Darmzustands.

## Revendications

1. Composition d'enrobage comprenant un milieu aqueux et, dispersé dedans, de l'amidon qui comprend au moins 40 % d'amylose et un agent filmogène qui est un polymère sensiblement insoluble dans l'eau qui contrôle le gonflement de l'amidon par le milieu aqueux.

2. Composition selon la revendication 1, dans laquelle le milieu aqueux est de l'eau, ou comprend de l'eau et un alcool (par exemple, éthanol).

3. Composition selon l'une quelconque des revendications précédentes, dans laquelle l'amidon comprend de 40 à 80 % d'amylose.

4. Composition selon l'une quelconque des revendications précédentes, dans laquelle l'amidon comprend de 50 à 70 % d'amylose.

5. Composition selon l'une quelconque des revendications précédentes, dans laquelle l'agent filmogène inhibe le gonflement de l'amidon par le milieu aqueux.

6. Composition selon l'une quelconque des revendications précédentes, dans laquelle l'agent filmogène comprend de l'éthylcellulose.

7. Composition selon l'une quelconque des revendications précédentes, dans laquelle le rapport entre amidon et agent filmogène est compris entre 1:1 et 1:6.

8. Composition selon l'une quelconque des revendications précédentes, qui comprend en outre un plastifiant, dans laquelle en option le plastifiant est du sébacate de dibutyle.

9. Formulation pour administration de médicament dans le côlon comprenant un noyau comprenant un principe actif et un enrobage qui comprend de l'amidon qui comprend au moins 40 % d'amylose et un agent filmogène qui est un polymère sensiblement insoluble dans l'eau qui contrôle le gonflement de l'amidon par un milieu aqueux.

10. Formulation selon la revendication 9 qui peut être obtenue en enrobant un noyau comprenant un principe actif d'une composition selon l'une quelconque des revendications 1 à 8.

11. Formulation selon la revendication 9 ou 10, dans laquelle le principe actif est un produit pharmaceutique, un vaccin, une protéine ou un peptide, dans laquelle en option ledit principe actif est un agent de diagnostic.

12. Formulation selon l'une quelconque des revendications 9 à 11, qui comprend en outre un enrobage gastrorésistant externe, dans laquelle en option l'enrobage gastrorésistant comprend un copolymère pouvant être obtenu par polymérisation de monomères y compris d'acide méthacrylique et d'acrylate d'éthyle.

13. Formulation selon l'une quelconque des revendications 9 à 12, pour une utilisation en thérapie ou diagnostic, et en option pour une utilisation dans la thérapie ou le diagnostic d'une maladie ou d'un problème de côlon.

14. Formulation selon l'une quelconque des revendications 9 à 13, qui est une formulation à libération contrôlée.

15. Formulation selon l'une quelconque des revendications 9 à 14, dans laquelle l'agent actif est un aminosalicylate ou un corticostéroïde.

16. Procédé de production d'une composition selon l'une quelconque des revendications 1 à 8, qui comprend l'étape consistant à mélanger un milieu aqueux, de l'amidon qui comprend au moins 40 % d'amylose et un agent filmogène qui est un polymère sensiblement insoluble dans l'eau qui contrôle le gonflement de l'amidon par le milieu aqueux, dans lequel en option le procédé comprend l'étape consistant à mélanger une dispersion comprenant de l'amidon avec une dispersion contenant ledit agent filmogène.

17. Procédé selon la revendication 16, dans lequel le procédé comprend en outre l'étape consistant à enrober une formulation avec ladite composition.

18. Formulation selon la revendication 15 à utiliser dans la thérapie ou le diagnostic d'une maladie ou d'un problème de côlon.
